# EUROPÄISCHE PATENTSCHRIFT

(11) **EP 1 115 332 B1**
(45) Veröffentlichungstag und Bekanntmachung des Hinweises auf die Patenterteilung: **21.04.2004**
(21) Anmeldenummer: 99948788.7
(22) Anmeldetag: 18.09.1999
(51) Int. Cl.: A61B 5/107

(54) **HAARLÄNGENMESSGERÄT**
APPARATUS FOR MEASURING HAIR LENGTH
APPAREIL DE MESURE DE LA LONGUEUR DES CHEVEUX

(30) Priorität: 25.09.1998 DE 19844042
(43) Veröffentlichungstag der Anmeldung: 18.07.2001
(73) Patentinhaber: Panz, Ulla-Monika, 71566 Althütte (DE)
(72) Erfinder: Panz, Ulla-Monika, 71566 Althütte (DE)
(74) Vertreter: Wolf, Eckhard, Dr.-Ing.
(86) Internationale Anmeldenummer: PCT/EP1999/006926
(87) Internationale Veröffentlichungsnummer: WO 2000/018297

(56) Entgegenhaltungen:
- DE-C- 613 009
- US-A- 1 508 811
- US-A- 3 377 712

## Beschreibung

Die Erfindung betrifft eine Vorrichtung zur Messung der Länge des Haupthaares und/oder der Gesichtsproportionen eines Probanden.

Untersuchungen haben ergeben, daß die Proportionen des Gesichts und des Körpers eines Probanden mit bestimmten Haarlängen besonders gut harmonieren, während andere Haarlängen einen eher unproportionierten Eindruck erwekken. Ein Friseur oder Stylist mußte sich in der Vergangenheit bei der Frisurberatung auf sein Gefühl verlassen, um die für den jeweiligen "Typ" eines Probanden die passende Frisur zu bestimmen.

Dokument US-A-1 508 811 offenbart eine Vorrichtung zur Messung der Dimensionen des Kopfs.

Die Aufgabe der Erfindung besteht daher darin, eine Vorrichtung bereitzustellen, mit der die Haarlänge und die Gesichtsproportionen eines Probanden genau und reproduzierbar bestimmt werden können.

Die Erfindung geht vor allem von dem Gedanken aus, daß die Haarlänge und die Gesichtsproportionen besonders dann genau und reproduzierbar bestimmt werden können, wenn die Messung von einem definierten Bezugspunkt aus erfolgt. Erfindungsgemäß ist daher ein auf das Haupt aufsetzbares, ein im Bereich der Scheitelhöhe angeordnetes, flexibles Meßorgan aufweisendes Gestell vorgesehen. Das Gestell besteht im wesentlichen aus einem in Längsrichtung des Hauptes verlaufenden ersten Haltebügel und einem quer dazu angeordneten zweiten Haltebügel aus einem elastischen Material, die sich im Bereich der Scheitelhöhe kreuzen. Um ein bequemes Aufsetzen des Gestells auf das Haupt zu ermöglichen, sollten die Haltebügel an die Kontur des Hauptes angepaßt sein.

Der sich in Längsrichtung des Hauptes verlaufende erste Haltebügel erstreckt sich vorteilhaft vom Nackenbereich bis in den Stirnbereich, während der dazu quer angeordnete zweite Haltebügel auf beiden Seiten des Hauptes oberhalb der Ohren endet. Das Meßorgan ist dabei vorzugsweise am Kreuzungspunkt der Haltebügel angeordnet.

Die Haarlänge und die Gesichtproportionen des Probanden lassen sich auf besonders einfache und genaue Weise bestimmen, wenn das Meßorgan als vorzugsweise eine Zentimeter- und/oder Millimeterskalierung aufweisendes Maßband ausgebildet ist.

Das Gestell besteht vorzugsweise aus vorgeformten Kunststoff- oder Blechstreifen, die mittels einer Kleb- oder Nietverbindung miteinander verbunden sind. Um eine Messung in jeder gewünschten Richtung vornehmen zu können, sollte das Meßorgan um eine vertikale Achse frei drehbar an dem Gestell befestigt sein.

Im folgenden wird die Erfindung anhand eines in der Zeichnung schematisch dargestellten Ausführungsbeispiels näher erläutert und beschrieben. Es zeigt:

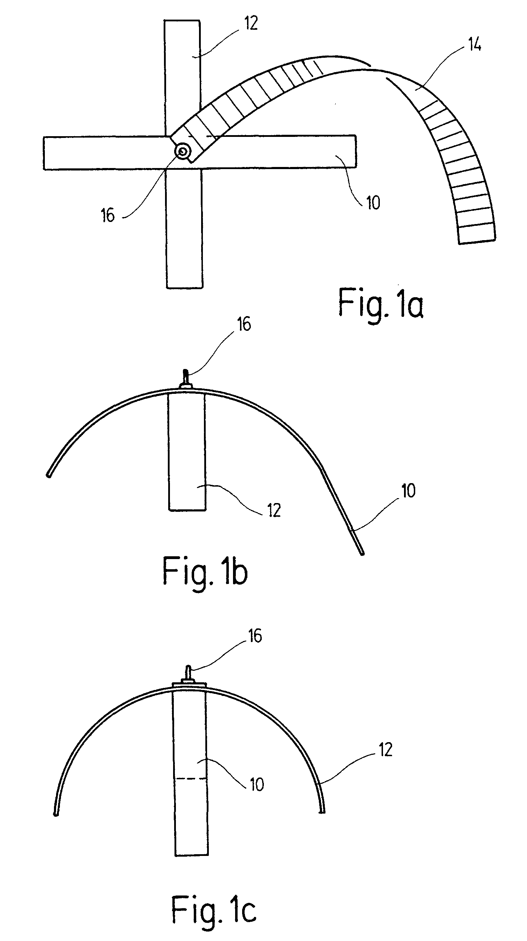

- Fig. 1a: eine Ansicht von oben auf die erfindungsgemäße Vorrichtung;
- Fig. 1b: eine Seitenansicht des Gestells der Vorrichtung gemäß Fig. 1a; und
- Fig. 1c: eine Vorderansicht des Gestells der Vorrichtung gemäß Fig. 1a.

Die in der Zeichnung schematisch dargestellte Vorrichtung dient zur Bestimmung der Haarlänge und/oder der Gesichtsproportionen eines Probanden. Die Vorrichtung besteht im wesentlichen aus einem aus zwei Haltebügeln 10, 12 bestehendem Gestell und einem als Maßband ausgebildetem Meßorgan 14, wobei die Haltebügel 10, 12 einander in einem rechten Winkel kreuzen und mittels einer Kleb- oder Nietverbindung miteinander verbunden sind. Die Haltebügel bestehen aus elastischem Kunststoff oder Metall, so daß sich das Gestell helmartig auf das Haupt des Probanden aufsetzen läßt und dort sicher anliegt, ohne unangenehm zu drücken. Der Tragekomfort wird noch dadurch gesteigert, daß die Haltebügel 10, 12 anatomisch vorgeformt sind. Der in Längsrichtung verlaufende Haltebügel 10 erstreckt sich etwa vom Stirnbereich bis in den Nackenbereich, während der dazu quer angeordnete Haltebügel 12 auf beiden Seiten des Hauptes oberhalb der Ohren endet.

Der Kreuzungspunkt der Haltebügel 10, 12 befindet sich im Bereich der Scheitelhöhe des Hauptes. An dieser Stelle ist auch das Maßband 14 um eine vertikale Achse frei drehbar an einem Stift 16 befestigt. Der Stift 16 bzw. die Scheitelhöhe bildet damit den Bezugspunkt, von dem aus die Haarlänge des Probanden sowie dessen Gesichtsproportionen vermessen werden. Untersuchungen haben ergeben, daß für bestimmte Gesichtsproportionen, d.h. Abstände der Augen, der Nase, des Mundes und des Kinns voneinander bzw. von dem Bezugspunkt 16, die optimale, d.h. einen besonders positiven Gesamteindruck ergebende, Haarlänge nach einem mathematischen Verfahren berechenbar ist.

Zusammenfassend ist folgendes festzustellen: Die Erfindung betrifft eine Vorrichtung zur Messung der Länge des Haupthaares und/oder der Gesichtsproportionen eines Probanden. Um eine genaue und reproduzierbare Messung zu ermöglichen, ist gemäß der Erfindung ein auf das Haupt aufsetzbares, ein im Bereich der Scheitelhöhe angeordnetes, flexibles Meßorgan aufweisendes Gestell vorgesehen, welches aus zwei quer zueinander angeordneten, flexiblen Haltebügeln 10, 12 besteht, an dessen Kreuzungspunkt 16 das als Maßband ausgebildete Meßorgan 14 frei drehbar befestigt ist.

## Patentansprüche

1. Vorrichtung zur Messung der Länge des Haupthaares und/oder der Gesichtsproportionen eines Probanden mit einem auf das Haupt aufsetzbaren Gestell, das zwei quer zueinander verlaufende, sich im Bereich der Scheitelhöhe kreuzende Haltebügel (10,12) aufweist **gekennzeichnet durch** ein am Kreuzungspunkt (16) drehbar befestigtes flexibles Meßorgan (14).

2. Vorrichtung nach Anspruch 1, **dadurch gekennzeichnet, daß** einer der Haltebügel (10) in Längsrichtung des Hauptes und der andere Haltebügel (12) quer dazu verläuft.

3. Vorrichtung nach Anspruch 1 oder 2, **dadurch gekennzeichnet, daß** die Haltebügel aus elastischem Material bestehen.

4. Vorrichtung nach einem der Ansprüche 1 bis 3, **dadurch gekennzeichnet, daß** die Haltebügel (10, 12) an die Kontur des Hauptes angepaßt sind.

5. Vorrichtung nach einem der Ansprüche 2 bis 4, **dadurch gekennzeichnet, daß** sich der in Längsrichtung des Hauptes verlaufende Haltebügel (10) vom Nackenbereich bis in den Stirnbereich erstreckt.

6. Vorrichtung nach einem der Ansprüche 2 bis 5, **dadurch gekennzeichnet, daß** der zweite Haltebügel (12) auf beiden Seiten des Hauptes oberhalb der Ohren endet.

7. Vorrichtung nach einem der Ansprüche 1 bis 6, **dadurch gekennzeichnet, daß** das Meßorgan (14) als vorzugsweise eine Zentimeterskalierung aufweisendes Maßband ausgebildet ist.

8. Vorrichtung nach einem der Ansprüche 1 bis 7, **dadurch gekennzeichnet, daß** das Gestell (10, 12) aus vorgeformten Kunststoff- oder Blechstreifen besteht.

9. Vorrichtung nach Anspruch 8, **dadurch gekennzeichnet, daß** die Kunststoff- oder Blechstreifen mittels einer Kleb- oder Nietverbindung miteinander verbunden sind.

10. Vorrichtung nach einem der Ansprüche 1 bis 9, **dadurch gekennzeichnet, daß** das Meßorgan (14) um eine vertikale Achse frei drehbar an dem Gestell befestigt ist.

## Claims

1. Device for measuring the length of the hair of the head and/or the facial proportions of a subject, with a support seatable upon the head, which has two support brackets (10, 12) crossing each other in the region of the top of the head and extending perpendicularly to each other, **characterized by** a flexible measuring element (14) rotatably secured in the crossing point (16).

2. Device according to Claim 1, **characterized in that** one of the frame pieces (10) extends in the longitudinal direction of the head and the other frame piece (12) is oriented perpendicularly thereto.

3. Device according to Claim 1 or 2, **characterized in that** the frame piece is comprised of an elastic material.

4. Device according to one of Claims 1 through 3, **characterized in that** the frame pieces (10, 12) conform to the contour of the head.

5. Device according to one of Claims 2 through 4, **characterized in that** the frame piece (10) extending in the longitudinal direction of the head extends from the nape of the neck to the forehead.

6. Device according to one of Claims 2 through 5, **characterized in that** the second frame piece (12) ends on both sides of the head above the ears.

7. Device according to one of Claims 1 through 6, **characterized in that** the measuring element (14) is a measuring tape preferably having centimeter gradations.

8. Device according to one of Claims 1 through 7, **characterized in that** the support (10, 12) is comprised of pre-formed plastic or metal strips.

9. Device according to Claim 8, **characterized in that** the plastic or metal strips are connected to each other via adhesive or rivet connections.

10. Device according to one of Claims 1 through 9, **characterized in that** the measuring means (14) is secured to the support freely rotatable about a vertical axis.

## Revendications

1. Dispositif pour mesurer la longueur des cheveux et/ou les proportions du visage d'un sujet, avec un support qui peut être placé sur la tête et qui présente deux étriers de maintien (10, 12) s'étendant transversalement l'un par rapport à l'autre, se croisant dans la région du sommet de la tête, **caractérisé par** un organe de mesure flexible (14) fixé à rotation au point d'intersection (16).

2. Dispositif selon la revendication 1, **caractérisé en ce qu'**un (10) des étriers de maintien s'étend dans la direction longitudinale de la tête, et l'autre étrier de maintien (12) transversalement au précédent.

3. Dispositif selon la revendication 1 ou 2, **caractérisé en ce que** les étriers de maintien sont réalisés en matériau élastique.

4. Dispositif selon l'une des revendications 1 à 3, **caractérisé en ce que** les étriers de maintien (10, 12) sont adaptés au contour de la tête.

5. Dispositif selon l'une des revendications 2 à 4, **caractérisé en ce que** l'étrier de maintien (10) s'étendant dans la direction longitudinale de la tête s'étend depuis la région du cou jusque dans la région du front.

6. Dispositif selon l'une des revendications 2 à 5, **caractérisé en ce que** le deuxième étrier de maintien (12) se termine de part et d'autre de la tête au-dessus des oreilles.

7. Dispositif selon l'une des revendications 1 à 6, **caractérisé en ce que** l'organe de mesure (14) est réalisé sous forme de mètre ruban présentant de préférence une graduation en centimètres.

8. Dispositif selon l'une des revendications 1 à 7, **caractérisé en ce que** le support (10, 12) est constitué de bandes préformées de matière plastique ou de tôle.

9. Dispositif selon la revendication 8, **caractérisé en ce que** les bandes de matière plastique ou de tôle sont mutuellement reliées par collage ou rivetage.

10. Dispositif selon l'une des revendications 1 à 9, **caractérisé en ce que** l'organe de mesure (14) est fixé sur le support à libre rotation autour d'un axe vertical.
